(19) Europäisches Patentamt
European Patent Office
Office européen des brevets

(11) EP 1 756 004 B1

(12) EUROPEAN PATENT SPECIFICATION

(45) Date of publication and mention of the grant of the patent:
**18.08.2010 Bulletin 2010/33**

(21) Application number: **04822021.4**

(22) Date of filing: **23.04.2004**

(51) Int Cl.:
***C01B 39/00*** *(2006.01)*

(86) International application number:
**PCT/US2004/012831**

(87) International publication number:
**WO 2005/113437 (01.12.2005 Gazette 2005/48)**

(54) **HIGH SILICA ZEOLITES UZM-5HS**

SILICIUMREICHE ZEOLITHE UZM-5HS

ZEOLITES UZM-5HS A FORTE TENEUR EN SILICE

(84) Designated Contracting States:
**DE FR IT**

(43) Date of publication of application:
**28.02.2007 Bulletin 2007/09**

(73) Proprietor: **UOP LLC**
**Des Plaines, IL 60016-6100 (US)**

(72) Inventors:
- **JAN, D. Y.;**
  **c/o UOP LLC**
  **Des Plaines, IL 60016 (US)**
- **MOSCOSO, Jaime G.;**
  **c/o UOP LLC**
  **Des Plaines, IL 60016 (US)**
- **LEWIS, G. J.;**
  **c/o UOP LLC**
  **Des Plaines, IL 60016 (US)**
- **GATTER, M. G.;**
  **c/o UOP LLC**
  **Des Plaines, IL 60016 (US)**
- **MEZZA, B. J.;**
  **c/o UOP LLc**
  **Des Plaines, IL 60016 (US)**
- **KOSTER, S. C.;**
  **c/o UOP LLc**
  **Des Plaines, IL 60016 (US)**

(74) Representative: **Chung, Hsu Min**
**Boult Wade Tennant**
**Verulam Gardens**
**70 Gray's Inn Road**
**London WC1X 8BT (GB)**

(56) References cited:
**EP-A- 0 082 211**
**WO-A-2004/039725**
**US-A- 4 503 023**
**US-A- 4 996 034**
**US-A1- 2003 211 034**
**US-B1- 6 388 159**
**US-B1- 6 613 302**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 756 004 B1

Printed by Jouve, 75001 PARIS (FR)

## Description

This invention relates to a family of related crystalline aluminosilicate UZM-5HS zeolites, which are derived from UZM-5 zeolites. The aluminium content of the UZM-5HS is lower than that of the starting UZM-5 thus changing its ion exchange capacity and acidity.

Zeolites are crystalline aluminosolicate compositions which are microporous and which are formed from corner sharing $AlO_2$ and $SiO_2$ tetrahedra. Numerous zeolites, both naturally occurring and synthetically prepared are used in various industrial processes. Zeolites are characterized by having pore openings of uniform dimensions, having a significant ion exchange capacity, and being capable of reversibly desorbing an adsorbed phase which is dispersed throughout the internal voids of the crystal without significantly displacing any atoms which make up the permanent zeolite crystal structure.

The number of synthetic zeolites is well over a hundred as evidenced by *Atlas of Zeolite Framework Types* published by the Structure Commission of the International Zeolite Association (IZA). As it well known, zeolites are distinguished from each other on the basis of their composition, crystal structure, catalytic and absorption properties. One method commonly used in the art to distinguish zeolites is x-ray diffraction. US 4,996,034 discloses a process for substituting silicon into the crystal lattice of zeolites in place of extracted lattice aluminium atoms to achieve zeolite products of higher silicon content which comprises using a starting aluminosolicate zeolite having a framework $SiO_2/Al_2O_3$ molar ratio of at least 2 and pore diameters of at least 3 Angstroms.

US 4,503,023 discloses extracting and substituting aluminium from $AlO_4$-tetrahedra of as-synthesized zeolites with silicon to form zeolite compositions having higher $SiO_2/Al_2O_3$ molar ratios and exhibiting distinctive chemical and physical properties. The procedure involves contact of the starting zeolite with an aqueous solution of a fluorosilicate salt using controlled proportions and temperature and pH conditions which avoid aluminium extraction without silicon substitution.

US 2003/0211034 discloses a crystalline aluminosilicate zeolite identified as UZM-4M. This zeolite is obtained by treating a UZM-4 zeolite with a fluorosilicate salt to obtain a zeolite having the empirical formula:

$$M1_a^{n+}Al_{1-x}E_xSi_yO_z$$

Where M1 is an alkali metal, alkaline earth metal, rare earth metal, hydronium ion or ammonium ion, E can be gallium, iron, boron, indium and mixtures thereof and has a Si/Al ratio of 1.5 to about 10.

UZM-5 zeolites are a family of zeolites which are described in US 6,613,302 B1 and US 6,388,159 B1. The UZM-5 zeolitic compositions have a unique x-ray diffraction pattern and have an empirical formula on an anhydrous basis in terms of molar ratios of:

$$M^{n+}_mR_r^{p+}\ Al_{(1-x)}E_xSi_yO_z$$

where M is at least one exchangeable cation selected from the group consisting of alkali and alkaline eearth metals, m is the mole ratio of M to (A1 + E) and varies from 0 to 1.2, R is a nitrogen-containing organic cation selected from the group consisting of quaternary ammonium ions, protonated amines, protonated diamines, protonated alkanolamines, quaternary alkanolammonium ions, diquaternary ammonium ions, and mixtures thereof, r is the mole ratio of R to (Al + E) and has a value of 0.25 to 3.0, E is an element selected from the group consisting of Ga, Fe, and B, x is the mole fraction of E and varies from 0 to 0.5, n is the weighted average valence of M and has a value of +1 to +2, p is the weighted average value of R and has a value of +1 to +2, y is the mole ratio of Si to (Al + E) and varies from 5 to 12, and z is the mole ratio of O to Al and has a value determined by the equation:

$$z = (m \bullet n + r \bullet p + 3 + 4 \bullet y)/2$$

Specific members of this family of zeolites are UZM-5 and UZM-5P.

Applicants have now modified these UZM-5 materials in order to change some of their properties. By using one or more of acid extracting, calcination, steaming and ammonium hexafluorosilicate treatment, applicants have been able to control the aluminum content of the UZM-5 zeolites to nearly all silica while maintaining their structure and porosity. Control of the Al composition in the zeolite allows one to tune the properties associated with the Al, such as

ion-exchange capacity and acidity thereby providing improved catalysts and/or adsorbents. This new family of materials are designated UZM-5HS.

## DETAILED DESCRIPTION OF THE INVENTION

In a first aspect, the present invention provides a microporous crystalline zeolite having a three-dimensional framework of at least $AlO_2$ and $SiO_2$ tetrahedral oxide units and an empirical composition on an anhydrous basis in terms of mole ratios of the elements of:

$$M1_a^{n+}Al_{(1-x)}E_xSi_{y'}O_{z''}$$

where M1 is at least one exchangeable cation selected from the group consisting of alkali metals, alkaline earth metals, rare earth metals, ammonium ion, hydrogen ion and mixtures thereof, a is the mole ratio of M1 to (Al + E) and varies from 0.15 to 5.0, E is an element selected from the group consisting of gallium, iron, boron, chromium, indium and mixtures thereof, x is the mole fraction of E and varies from 0 to 0.5, n is the weighted average valence of M1 and has a value of +1 to +3, y' is the mole ratio of Si to (A1 + E) and is greater than 5 and z" is the mole ratio of O to (A1 + E) and has a value determined by the equation:

$$Z'' = (a \bullet n + 3 + 4 \bullet y')/2$$

the zeolite characterised in that it has at least two x-ray diffraction peaks, one at a *d*-spacing of 3.84±0.07Å and one at a *d*-spacing of 8.55±0.25Å, and further characterized in that it has an x-ray powder diffraction pattern which contains at least the d-spacing and relative intensities given in the Table below:

**Table A**

| **UZM-5HS** | | |
|---|---|---|
| 2-Θ | d(Å) | $I/I_0$% |
| <6.79 | >13.0 | w-m |
| 8.26 - 7.52 | 10.70 - 11.75 | m-vs |
| 10.65 - 10.04 | 8.30 - 8.80 | m-vs |
| 12.32 - 11.79 | 7.18 - 7.50 | s-vs |
| 16.56 - 15.53 | 5.35 - 5.70 | m-vs |
| 19.71 - 18.78 | 4.50 - 4.72 | w-m |
| 23.58 - 22.72 | 3.77 - 3.91 | w-m |
| 24.37 - 23.64 | 3.65 - 3.76 | m-vs |

In a second aspect the present invention provides a hydrocarbon conversion process comprising contacting a hydrocarbon steam with a catalytic composite at hydrocarbon conversion conditions to give a converted product, the catalytic composite comprising the zeolite as defined herein.

The aluminosilicate zeolites (UZM-5HS) of the invention and substituted versions of the same have a unique structure related to UZM-5. UZM-5HS is obtained by treating a starting zeolite having the topology of UZM-5 with: a) a fluorosilicate solution or slurry; b) calcination or steaming followed by acid extraction or ion-exchange; c) acid extraction or d) any combination of these processes in any order. UZM-5 zeolites are described in US 6,613,302 B1 and US 6,388,159B1. As described in the above references, UZM-5 has a composition in the as-synthesized form on an anhydrous basis expressed by the empirical formula:

$$M_m^{n+} R_r^{p+} Al_{(1-x)} E_x Si_y O_z \quad (1)$$

where M is at least one exchangeable cation and is selected from the group consisting of alkali and alkaline earth metals.

Specific examples of the M cations include but are not limited to lithium, sodium, potassium, cesium, strontium, calcium, magnesium, barium and mixtures thereof. The value of m which is the mole ratio of M to (Al + E) varies from 0 to 1.2. R is a nitrogen containing organic cation and is selected from the group consisting of protonated amines, protonated diamines, protonated alkanolamines, quaternary ammonium ions, diquaternary ammonium ions, quaternized alkanolammonium ions and mixtures thereof. The value of **r** which is the mole ratio of R to (Al + E) varies from 0.25 to 3.0. The value of **n** which is the weighted average valence of M varies from +1 to +2. The value of **p,** which is the average weighted valence of the organic cation has a value from +1 to +2. E is an element which is tetrahedrally coordinated, is present in the framework and is selected from the group consisting of gallium, iron, boron, chromium, indium, and mixtures thereof. The value of **x** which is the mole fraction of E varies from 0 to 0.5. The ratio of silicon to (Al+E) is represented by **y** which varies from 5 to 12, while the mole ratio of O to (Al+E) is represented by **z** and has a value given by the equation:

$$z = (m \bullet n + r \bullet p + 3 + 4 \bullet y)/2$$

When M is only one metal, then the weighted average valence is the valence of that one metal, i.e. +1 or +2. However, when more than one M metal is present, the total amount of

$$M_m^{n+} = M_{m1}^{(n1)+} + M_{m2}^{(n2)+} + M_{m3}^{(n3)+} + .....$$

and the weighted average valence n is given by the equation:

$$n = \frac{m_1 \bullet n_1 + m_2 \bullet n_2 + m_3 \bullet n_3 + \cdots}{m_1 + m_2 + m_3 \cdots}$$

Similarly when only one R organic cation is present, the weighted average valence is the valence of the single R cation, i.e., +1 or +2. When more than one R cation is present, the total amount of R is given by the equation.

$$R_r^{p+} = R_{r1}^{(p1)+} + R_{r2}^{(p2)+} + R_{r3}^{(p3)+}$$

and the weighted average valence p is given by the equation

$$p = \frac{p_1 \bullet r_1 + p_2 \bullet r_2 + p_3 \bullet r_3 + \cdots}{r_1 + r_2 + r_3 + \cdots}$$

These aluminosilicate zeolites, are prepared by a hydrothermal crystallization of a reaction mixture prepared by combining reactive sources of M, R, aluminum, and silicon in aqueous media. Accordingly, the aluminum sources include, but are not limited to, aluminum alkoxides, precipitated alumina, aluminum hydroxide, aluminum salts and aluminum metal. Specific examples of aluminum alkoxides include, but are not limited to aluminum sec-butoxide, and aluminum isopropoxide. Sources of silica include but are not limited to tetraethylorthosilicate, fumed silicas, precipitated silicas and colloidal silica. Sources of the M metals include the halide salts, nitrate salts, acetate salts, and hydroxides of the respective alkali or alkaline earth metals. When R is a quaternary ammonium cation, the sources include the hydroxide, carbonate, acetate and halide compounds. Specific examples include without limitation tetramethylammonium hydroxide, tetraethylammonium hydroxide, hexamethonium bromide, tetramethylammonium chloride, methyltriethylammonium hydroxide and tetramethylammonium carbonate. R may also be neutral amines, diamines, and alkanolamines. Specific examples are triethanolamine, triethylamine, and N,N,N',N' tetramethyl-1,6-hexanediamine.

The reaction mixture containing reactive sources of the desired components can be described in terms of molar ratios of the oxides by the formula:

$aM_{2/n}O:bR_{2/n}O:(1-c)Al_2O_3:cE_2O_3:dSiO_2:eH_2O$

where **a** is the mole ratio of the oxide of M and has a value of 0 to 2, **b** is the mole ratio of the oxide of R and has a value of 1.5 to 30, **d** is the mole ratio of silica and has a value of 5 to 30, c is the mole fraction of the oxide of E and has a value from 0 to 0.5, and **e** is the mole ratio of water and has a value of 30 to 6000. The reaction mixture is now reacted at a temperature of 100˚C to 175˚C and preferably from 120˚C to 150˚C for a period of 12 hours to 14 days and preferably for a time of 2 days to 6 days in a sealed reaction vessel under autogenous pressure. After crystallization is complete, the solid product is isolated from the heterogeneous mixture by means such as filtration or centrifugation, and then washed with de-ionized water and dried in air at ambient temperature up to 100˚C.

In this respect, the following species can be identified by their x-ray diffraction patterns having at least the *d*-spacing and relative intensities set forth in Tables B and C. As shown in the '860 application, the UZM-5 family of zeolites is characterized by having at least two peaks in the x-ray diffraction pattern: one peak at a *d*-spacing of 3.9 ± 0.12 Å and one peak at a *d*-spacing of 8.6 ± 0.20 Å. UZM-5P phases have been observed with peaks at *d*-spacing up to 40Å under certain growth conditions.

Table B

| UZM-5 | | |
|---|---|---|
| 2-Theta | d(Å) | $I/I_o$% |
| 6.31- 5.89 | 14.00-15.00 | m |
| 7.96- 7.58 | 11.10 - 11.65 | m-s |
| 10.40- 10.01 | 8.50 - 8.83 | w-m |
| 12.11 - 11.59 | 7.30-7.63 | m |
| 16.10- 15.53 | 5.50 - 5.70 | m-vs |
| 19.28 - 18.55 | 4.60 - 4.78 | w-m |
| 22.26 - 21.60 | 3.99 - 4.11 | m |
| 23.20 - 22.43 | 3.83 - 3.96 | w-s |
| 24.16 - 23.33 | 3.68 - 3.81 | vs |
| 30.48 - 29.55 | 2.93 - 3.02 | w-m |
| 31.94 - 30.92 | 2.80 - 2.89 | w-m |
| 44.83 - 43.47 | 2.02 - 2.08 | w |

Table C

| UZM-5P | | |
|---|---|---|
| 2-Theta | d(Å) | $I/I_o$% |
| <6.31 | >14.00 | w - vs |
| 7.96 - 7.68 | 11.10 - 11.50 | w - m |
| 10.52-10.04 | 8.40 - 8.80 | m - s |
| 16.56 - 15.67 | 5.35 - 5.65 | w - m |
| 19.49 - 18.87 | 4.55 - 4.70 | w - m |
| 23.52 - 22.09 | 3.78 - 4.02 | w - vs |
| 24.03 - 23.39 | 3.70 - 3.80 | w - m |
| 30.81 - 29.76 | 2.90 - 3.00 | w - m |
| 31.94 - 30.81 | 2.80-2.90 | w - m |
| 45.30 - 43.04 | 2.00 - 2.10 | w - m |

The cation population of the starting UZM-5 is not a critical factor of the instant process insofar as the dealumination processes are concerned, but can have a bearing on the final result, especially with regard to the extent of dealumination. Thus, the UZM-5 can be used as synthesized or can be ion exchanged to provide a different cation form. In this respect, the starting zeolite can be described by the empirical formula:

$$M'^{n'+}_{m'} R^{p+}_{r'} Al_{(1-x)} E_x Si_y O_{z'} \qquad (2)$$

where R, **x, y,** and E are as described above and **m'** has a value from 0 to 3.0, M' is a cation selected from the group consisting of alkali metals, alkaline earth metals, rare earth metals, hydrogen ion, ammonium ion, and mixtures thereof, **n'** is the weighted average valence of M' and varies from 1 to 3, **r'** has a value from 0 to 3.0, and **p** is the weighted average valence of R and varies from +1 to +2. The value of **z'** is given by the formula:

$$z' = (m' \bullet n' + r' \bullet p + 3 + 4 \bullet y)/2.$$

The designation UZM-5 will be used to refer to the zeolite represented by formula (2) which includes both the as-synthesized and ion exchanged forms of the zeolite.

Methods used to exchange one cation for another are well known in the art and involve contacting the microporous compositions with a solution containing the desired cation (at molar excess) at exchange conditions. Exchange conditions include a temperature of 15°C to 100°C and a time of 20 minutes to 50 hours. The organic cation can also be removed prior to ion exchange by heating under controlled conditions. A special case of ion-exchange is ammonia calcination, in which the organic template can be decomposed and replaced by ammonium cation.

In a preferred case, especially for dealumination by treatment with a fluorosilicate solution, the UZM-5 is exchanged with ammonium cation by contacting it with ammonium nitrate at a temperature of 15°C to 100°C, followed by a water wash. This procedure may be repeated several times. Finally, the exchanged UZM-5 zeolite is dried at 100°C.

One process of preparing the UZM-5HS of the present invention is by treating the UZM-5 composition described above with a fluorosilicate salt at a temperature of 20°C to 90°C. The fluorosilicate salt serves two purposes. It removes aluminum atoms from the framework and provides a source of extraneous silicon, which can be inserted into the framework (replacing the aluminum). The fluorosilicate salts which can be used are described by the general formula:

$$A_{2/n}SiF_6$$

where n is the valence of A and A is a cation selected from the group consisting of $NH_4^+$, $H^+$, $Mg^{2+}$, $Li^+$, $Na^+$, $Ba^{2+}$, $Cd^{2+}$, $Cu^+$, $Cu^{2+}$, $Ca^{2+}$, $Cs^+$, $Fe^{2+}$, $Ca^{2+}$, $Pb^{2+}$, $Mn^{2+}$, $Rb^+$, $Ag^+$, $Sr^{2+}$, $Tl^+$, and $Zn^{2+}$. The ammonium fluorosilicate is most preferred because of its substantial solubility in water and because it forms water soluble by-product salts upon reaction with the zeolite, namely $(NH_4)_3AlF_6$.

The fluorosilicate salt is contacted with the UZM-5 zeolite in the form of an aqueous solution or slurry at a pH in the range of 3 to 7. This solution is contacted with the zeolite either incrementally or continuously at a slow rate such that a sufficient proportion of the framework aluminum atoms removed are replaced by silicon atoms to retain at least 50%, preferably at least 70% of the framework (crystalline) structure of the starting UZM-5 zeolite. The amount of fluorosilicate necessary to carry out the process of this invention can vary considerably, but should be at least in an amount of 0.0075 moles of the fluorosilicate salt per 100 grams of starting zeolite. Once the reaction is complete, the product zeolite UZM-5HS is isolated by conventional techniques such as filtration.

Without wishing to be bound to any particular theory, the process of removing aluminum and inserting the silicon appears to proceed in two steps in which the aluminum extraction step will, unless controlled, proceed very rapidly while the silicon insertion is relatively slow. If dealumination becomes too extensive without silicon substitution, the crystal structure becomes seriously degraded and ultimately collapses. In general, the rate of aluminum extraction is decreased as the pH of the fluorosilicate solution in contact with the zeolite is increased within the range of 3 to 7 and as the concentration of the fluorosilicate in the reaction system is decreased. At pH values below 3, crystal degradation can be unduly severe, whereas at pH values higher than 7, silicon insertion is unduly slow. Also, increasing the reaction temperature tends to increase the rate of substitution of silicon. Increasing the reaction temperature has been found to have less of an effect on dealumination than the pH of the solution. Therefore, the pH may be considered a means of controlling the dealumination while temperature may be considered as a means of controlling the substitution rate.

Theoretically, there is no lower limit for the concentration of fluorosilicate salt in the aqueous solution employed,

provided, of course, the pH of the solution is high enough to avoid undue destructive attack on the UZM-5 zeolite structure apart from the intended reaction with the fluorosilicate. A slow rate of addition of fluorosilicate salts insures that adequate time is permitted for the insertion of silicon into the framework before excessive aluminum extraction occurs with consequent collapse of the crystal structure. In general the effective reaction temperature is between 10˚C and 99˚C, preferably between 20˚C and 95˚C, but temperatures of 125˚C or higher and as low as 0˚C can be used.

The maximum concentration of fluorosilicate salt in the aqueous solution employed is, of course, interrelated to the temperature and pH factors and also with the time of contact between the zeolite and the solution and the relative proportions of zeolite and fluorosilicate salt. Solutions having fluorosilicate salt concentrations of between $10^{-3}$ moles per liter of solution and up to saturation of the solution can be employed, but it is preferred that concentrations in the range of between 0.05 and 2.0 moles per liter of solution be used. In addition, as hereinbefore discussed, slurries of the fluorosilicate salts may be employed. The aforementioned concentration values are with respect to true solutions, and are not intended to apply to the total fluorosilicate salts in slurries of the salts in water. Even very slightly soluble fluorosilicate salts can be slurried in water and used as a reagent, the undissolved solids being readily available to replace dissolved molecular species consumed in reaction with the zeolite. The minimum value for the amount of fluoro salt to be added is preferably at least equivalent to the minimum mole fraction of aluminum to be removed from the zeolite.

It has been found that when large amounts of silicon atoms are to be substituted, i.e., increasing the $SiO_2/Al_2O_3$ ratio by more than 100%, it is preferable to carry out the process in multiple steps in order to minimize crystal degradation. As the amount of silicon that is substituted into the framework is substantially increased (beyond 100% increase) it may actually be necessary to carry out the process in two or more steps in order to prevent excessive degradation of the crystalline structure. That is, contacting with the fluorosilicate salt is carried out in two or more steps using a lower concentration of the fluorosilicate salt than required to replace the desired amount of silicon in one step. After each fluorosilicate treatment, the product is washed to remove fluoride and aluminum. Drying of the zeolite at 50˚C between treatments may also be done to facilitate the handling of the wet zeolite product.

Another embodiment of the invention involves contacting the UZM-5 starting zeolite with an acid (acid extraction) in order to remove some of the aluminum from the framework and thereby provide the UZM-5HS zeolite of the invention. Although it is known that aluminum can be extracted from the framework by acids, it is not predictable whether the resulting product will retain a substantial portion of its crystallinity or whether the structure will collapse resulting in an amorphous material. Applicants have discovered that UZM-5 can be dealuminated to nearly pure silica forms while maintaining substantial crystallinity, surface area and micropore volume.

The acids which can be used in carrying out acid extraction include without limitation mineral acids, carboxylic acids and mixtures thereof. Examples of these include sulfuric acid, nitric acid, ethylene diaminetetraacetic acid (EDTA), citric acid, oxalic acid, etc. The concentration of acid which can be used is not critical but is conveniently between 1 wt. % to 80 wt.% acid and preferably between 5 wt.% and 40 wt.% acid. Acid extraction conditions include a temperature of 10˚C to 100˚C for a time of 10 minutes to 24 hours. Once treated with the acid, the UZM-5HS zeolite is isolated by means such as filtration, washed with deionized water and dried at ambient temperature up to 100˚C.

The extent of dealumination obtained from acid extraction depends on the cation form of the starting UZM-5 as well as the acid concentration and the time and temperature over which the extraction is conducted. For example, if organic cations are present in the starting UZM-5, the extent of dealumination will be slight compared to a UZM-5 in which the organic cations have been removed. This may be preferred if it is desired to have dealumination just at the surface of the UZM-5. Convenient ways of removing the organic cations include calcination, ammonia calcination, steaming and ion exchange. Calcination conditions include a temperature of 300˚C to 600˚C for a time of 2 to 24 hours. Steaming conditions include a temperature of 400˚C to 850˚C with from 1 % to 100% steam for a time of 10 minutes to 48 hours and preferably a temperature of 500˚C to 600˚C, steam concentration of 5 to 50% and a time of 1 to 2 hours. Ion exchange conditions are as set forth above.

A special treatment for removing organic cations to obtain the ammonium ion exchanged form is ammonia calcination. Calcination in an ammonia atmosphere can decompose organic cations, presumably to a proton form that can be neutralized by ammonia to form the ammonium cation. The stability of the ammonium form of the zeolite prevents dealumination upon hydration, which occurs extensively in lower ratio zeolites in the proton forms obtained in air calcinations. The resulting ammonium form of the zeolite can be further ion-exchanged to any other desired form. Ammonia calcination conditions include treatment in the ammonia atmosphere at temperatures between 250˚C and 600˚C and more preferably between 250˚C and 450˚C for times of 10 minutes to 5 hours. Optionally, the treatments can be carried out in multiple steps within this temperature range such that the total time in the ammonia atmosphere does not exceed 5 hours. Above 500˚C, the treatments should be brief, less than a half hour and more preferably on the order of 5-10 minutes. Extended calcination times above 500˚C can lead to unintended dealumination along with the desired ammonium ion-exchange and are unnecessarily harsh as most organoammonium templates easily decompose at lower temperatures.

It should be pointed out that both calcination and steaming treatments not only remove organic cations, but can also dealuminate the zeolite. Thus, alternate embodiments of the invention include: a calcination treatment followed

by acid extraction and steaming followed by acid extraction. A further embodiment of the invention comprises calcining or steaming the starting UZM-5 zeolite followed by an ion-exchange treatment. Of course an acid extraction can be carried out concurrently with, before or after the ion exchange.

The ion exchange conditions are the same as set forth above, namely a temperature of 15˚C to 100˚C and a time of 20 minutes to 50 hours. Ion exchange can be carried out with a solution comprising a cation (M1') selected from the group consisting of alkali metals, alkaline earth metals, rare earth metals, hydrogen ion, ammonium ion, and mixtures thereof. By carrying out this ion exchange, the M1 cation is exchanged for a secondary or different M1' cation. In a preferred embodiment, the UZM-5HS composition after the steaming or calcining steps is contacted with an ion exchange solution comprising an ammonium salt. Examples of ammonium salts include but are not limited to ammonium nitrate, ammonium chloride, ammonium bromide, and ammonium acetate. The ammonium ion containing solution can optionally contain a mineral acid such as but not limited to nitric, hydrochloric, sulfuric and mixtures thereof. The concentration of the mineral acid is that amount necessary to give a ratio of $H^+$ to $NH_4^+$ of 0 to 1. This ammonium ion exchange aids in removing any debris present in the pores after the steaming and/or calcination treatments.

It is apparent from the foregoing that, with respect to effective process conditions, it is desirable that the integrity of the zeolite crystal structure be substantially maintained throughout the dealumination process, and that the zeolite retains at least 50%, preferably at least 70 and more preferably at least 90% of its original crystallinity. A convenient technique for assessing the crystallinity of the products relative to the crystallinity of the starting material is the comparison of the relative intensities of the *d*-spacing of their respective X-ray powder diffraction patterns. The sum of the peak intensities, in arbitrary units above the background, of the starting material is used as the standard and is compared with the corresponding peak intensities of the products. When, for example, the numerical sum of the peak heights of the molecular sieve product is 85 percent of the value of the sum of the peak intensities of the starting zeolite, then 85 percent of the crystallinity has been retained. In practice it is common to utilize only a portion of the peaks for this purpose, as for example, five or six of the strongest peaks. Other indications of the retention of crystallinity are surface area and adsorption capacity. These tests may be preferred when the substituted metal significantly changes, e.g., increases, the absorption of x-rays by the sample or when peaks experience substantial shifts such as in the dealumination process.

After having undergone any of the dealumination treatments as described above, the UZM-5HS is usually dried and can be used in various processes as discussed below. Applicants have found the properties of the UZM-5HS can be further modified by one or more additional treatment. These treatments include steaming, calcining or ion exchanging and can be carried out individually or in any combination. Some of these combinations include but are not limited to:

steam → calcine → ion exchange;

calcine → steam → ion exchange;

ion exchange → calcine → steam

ion exchange → steam → calcine;

steam → calcine;

calcine → steam, etc.

The dealumination treatment described above can be combined in any order to provide the zeolites of the invention although not necessarily with equivalent result. It should be pointed out that the particular sequence of treatments, e.g., AFS, acid extraction, steaming, calcining, etc can be repeated as many times as necessary to obtain the desired properties. Of course one treatment can be repeated while not repeating other treatments, e.g., repeating the AFS two or more times before carrying out steaming or calcining; etc. Finally, the sequence and/or repetition of treatments will determine the properties of the final UZM-5HS composition.

The UZM-5HS as prepared above is described by the empirical formula on an anhydrous basis of:

$$M1_a^{n+} Al_{(1-x)} E_x Si_{y'} O_{z''}$$

where M1 is at least one exchangeable cation selected from the group consisting of alkali, alkaline earth metals, rare earth metals, ammonium ion, hydrogen ion and mixtures thereof, **a** is the mole ratio of M1 to (Al + E) and varies from 0.15 to 5.0, n is the weighted average valence of M1 and has a value of +1 to +3, E is an element selected from the group consisting of gallium, iron, boron, chromium, indium and mixtures thereof, **x** is the mole fraction of E and varies

from 0 to 0.5, **y'** is the mole ratio of Si to (Al + E) and varies from greater than 5 to virtually (pure silica) and **z"** is the mole ratio of O to (Al + E) and has a value determined by the equation:

$$z'' = (a \bullet n + 3 + 4 \bullet y')/2$$

the zeolite characterized in that it has at least two x-ray diffraction peaks, one at a *d*-spacing of 3.84±0.07Å and one at 8.55 ±0.25Å. The former peak is often very broad. By virtually pure silica is meant that virtually all the aluminum and/or the E metals have been removed from the framework. It is well know that it is virtually impossible to remove all the aluminum and/or E metal. Numerically, a zeolite is virtually pure silica when **y'** has a value of at least 3,000, preferably 10,000 and most preferably 20,000. Thus, ranges for **y'** are from 5 to 3,000 preferably greater than 12 to 3,000; 5 to 10,000 preferably greater than 12 to 10,000 and 5 to 20,000 preferably greater than 12 to 20,000.

In specifying the proportions of the zeolite starting material or adsorption properties of the zeolite product and the like herein, the "anhydrous state" of the zeolite will be intended unless otherwise stated. The term "anhydrous state" is employed herein to refer to a zeolite substantially devoid of both physically adsorbed and chemically adsorbed water.

The UZM-5HS zeolite obtained after one or more of the above described treatments will have x-ray diffraction patterns which are different (and thus unique) from that of UZM-5. A list of the major peaks that are common to all the UZM-5HS materials is given in table A.

Table A

| UZM-5HS | | |
|---|---|---|
| 2-Θ | d(Å) | $I/I_0$ % |
| < 6.79 | > 13.0 | w-m |
| 8.26 - 7.52 | 10.70 - 11.75 | m-vs |
| 10.65 - 10.04 | 8.30 - 8.80 | m-vs |
| 12.32 - 11.79 | 7.18-7.50 | s-vs |
| 16.56 - 15.53 | 5.35 - 5.70 | m-vs |
| 19.71 - 18.78 | 4.50 - 4.72 | w-m |
| 23.58 - 22.72 | 3.77 - 3.91 | w-m |
| 24.37 - 23.64 | 3.65 - 3.76 | m-vs |

The zeolites of this invention are capable of separating mixtures of molecular species based on the molecular size (kinetic diameter) or on the degree of polarity of the molecular species. When the separation of molecular species is based on molecular size, separation is accomplished by the smaller molecular species entering the intracrystalline void space while excluding larger species. The kinetic diameters of various molecules such as oxygen, nitrogen, carbon dioxide, carbon monoxide and various hydrocarbons are provided in D.W. Breck, Zeolite Molecular Sieves, John Wiley and Sons (1974) p. 636. The separation of hydrocarbons based on molecular size is a preferred application.

The crystalline microporous compositions of the present invention either as synthesized or after calcination can be used as catalysts or catalyst supports in hydrocarbon conversion processes. Hydrocarbon conversion processes are well known in the art and include but are not limited to cracking, hydrocracking, alkylation of both aromatics and isoparaffins, isomerization of both aromatics and paraffins, polymerization, reforming, dewaxing, hydrogenation, dehydrogenation, transalkylation, naphtha cracking, ring opening, dealkylation, hydration, dehydration, hydrotreating, hydrodenitrogenation, hydrodesulfurization, methanation and syngas shift process. Specific reaction conditions and the types of feeds which can be used in these processes are set forth in US-A-4,310,440 and US-A-4,440,871 which are incorporated by reference. Preferred hydrocarbon conversion processes are alkylation of aromatics, isomerization of xylenes, naphtha cracking, conversion of oxygenates to olefins, ring opening, transalkylation, alkylation of isoparaffins and isomerization of ethylbenzene.

Other reactions may be catalyzed by these crystalline microporous compositions, including base-catalyzed side chain alkylation of alkylaromatics, aldol-condensations, olefin double bond isomerization and isomerization of acetylenes, alcohol dehydrogenation, and olefin dimerization and oligomerization. Some of the reaction conditions and types of feeds that can be used in these processes are set forth in US-A-5,015,796 and in H. Pines, THE CHEMISTRY OF CATALYTIC HYDROCARBON CONVERSIONS, Academic Press (1981) pp. 123-154 and references contained therein,

which are incorporated by reference.

The X-ray patterns presented in the following examples (and tables above) were obtained using standard X-ray powder diffraction techniques. The radiation source was a high-intensity X-ray tube operated at 45 kV and 35 ma. The diffraction pattern from the copper K-alpha radiation was obtained by appropriate computer based techniques. Flat compressed powder samples were continuously scanned at 2° (2θ) per minute from 2° to 70°(2θ). Interplanar spacings (d) in Angstrom units were obtained from the position of the diffraction peaks expressed as 2θ where θ is the Bragg angle as observed from digitized data. Intensities were determined from the integrated area of diffraction peaks after subtracting background, "$I_o$" being the intensity of the strongest line or peak, and "I" being the intensity of each of the other peaks.

As will be understood by those skilled in the art, the determination of the parameter 2θ is subject to both human and mechanical error, which in combination can impose an uncertainty of ±0.4 on each reported value of 2θ and up to ±0.5 on reported values for nanocrystalline materials. This uncertainty is, of course, also manifested in the reported values of the *d*-spacing, which are calculated from the θ values. This imprecision is general throughout the art and is not sufficient to preclude the differentiation of the present crystalline materials from each other and from the compositions of the prior art. In some of the X-ray patterns reported, the relative intensities of the *d*-spacing are indicated by the notations vs, s, m and w which represent very strong, strong, medium, and weak, respectively. In terms of 100 X $I/I_o$, the above designations are defined as w = 0-15; m = 15-60; s = 60-80 and vs = 80-100. In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

In order to more fully illustrate the invention, the following examples are set forth. It is to be understood that the examples are only by way of illustration and are not intended as an undue limitation on the broad scope of the invention as set forth in the appended claims.

Example 1 UZM-5 synthesis

Aluminum tri-sec-butoxide (95+%), 58.75 g, was dissolved in 836.34 g TEAOH (35%). Then 294.73 g colloidal silica (Ludox ™ AS-40, 40% $SiO_2$) was added along with 10.18 g de-ionized water. The reaction mixture was stirred vigorously for an hour and placed in several teflon bottles and aged overnight at 95°C. An analysis of the resulting aluminosilicate mixture showed it to contain 4.67% Si.

A 400 g portion of this aluminosilicate mixture was treated with a TMACI solution (9.41 g TMACI (97%) in 20.0 g deionized water) and homogenized for 20 minutes. The reaction mixture was placed in 6 teflon-lined autoclaves and digested for 96 hr at 150°C at autogenous pressures. The solid products were isolated by centrifugation, washed with de-ionized water and dried at 98°C.

The solid products from each autoclave were combined. Elemental analyses showed the Si/Al ratio to be 6.89. The BET surface area and micropore volume of the calcined material was 520 $m^2/g$ and 0.20cc/g. The powder x-ray diffraction pattern showed that the material was UZM-5. Characteristic lines of the diffraction pattern are shown in Table 1.

Table 1

| 2-Θ | d(Å) | $I/I_0$ % |
|---|---|---|
| 6.12 | 14.43 | m |
| 7.70 | 11.47 | m |
| 10.04 | 8.80 | m |
| 11.80 | 7.49 | m |
| 15.62 | 5.67 | m-s |
| 16.06 | 5.51 | m |
| 18.98 | 4.67 | m |
| 21.74 | 4.08 | m |
| 22.70 | 3.91 | m |
| 23.56 | 3.77 | vs |
| 25.08 | 3.55 | w |
| 26.12 | 3.41 | w |

(continued)

| 2-Θ | d(Å) | $I/I_0$ % |
|---|---|---|
| 27.14 | 3.28 | w |
| 29.62 | 3.01 | w-m |
| 29.98 | 2.98 | w |
| 31.26 | 2.86 | m |
| 32.98 | 2.71 | w |
| 34.32 | 2.61 | w |
| 43.94 | 2.06 | w |

Example 2

A 12 gram sample of UZM-5 prepared similarly as in Example 1 was calcined in a vertical tube furnace configured with an upflow of ammonia. The ammonia flow rate was 1.1 l/min while the furnace was ramped using a 5˚C/min ramp rate with 0.5 hr dwells at 300˚C, 350˚C and 450˚C and a 6 minute dwell at 500˚C or 550˚C. The sample was then cooled down to room temperature in the presence of flowing ammonia to make the Na, $NH_4$ UZM-5 form. The starting zeolite contained 100% tetrahedral Al as determined by NMR, 11.9% C, N/Al = 1.32 and Na/Al = 0.05, the nitrogen and carbon coming from the template. After the ammonia calcination procedure discussed above and employing a 500˚C treatment in the last step, the carbon level was reduced to 0.7%, N/Al = 1.02, Na/Al = 0.05, and the Al was shown to be nearly 100% tetrahedral by NMR. The resulting ammonium form of the zeolite maintains maximum ion-exchange capacity by keeping the aluminum in the framework, while also enhancing ion-exchange capability because the non-exchangeable template cations have been replaced by ammonium cations. From this composition, many variations of the zeolite are now attainable via ion-exchange before one begins the dealumination process.

Example 3

A 10 g portion of the material from example 1 was calcined in the following manner. Under a $N_2$ atmosphere, the temperature was ramped to 300˚C at 2˚C/min, held at 300˚C for 1.5 hr, ramped to 420˚C at 2˚C/min, held for 1.5 hr, and ramped to 520˚C/min at 2˚C/min, and held for 1 hr under $N_2$, followed by conversion of the atmosphere to air and another 5.5 hr at 520˚C. An acidic solution was prepared by diluting 2.4 g 98% $H_2SO_4$ in 80 g de-ionized water and heated to 75˚C. The calcined material was added to this solution and stirred for 2 hr at 75˚C. The product was isolated by filtration, washed with de-ionized water, and dried at 98˚C.

Elemental analysis showed the product to have a Si/Al ratio of 31.7, while $N_2$ adsorption measurements yielded a BET surface area of 391$m^2$/g and a micropore volume of 0.13 cc/g. An x-ray diffraction pattern indicated the material to be UZM-5HS. Characteristic lines in the diffraction pattern are shown in Table 2.

Table 2

| 2-Θ | d(Å) | $I/I_0$ % |
|---|---|---|
| 6.52 | 13.54 | m |
| 8.06 | 10.96 | m-s |
| 10.48 | 8.43 | s-vs |
| 12.18 | 7.26 | vs |
| 16.26 | 5.45 | m |
| 17.61 | 5.03 | w |
| 19.41 | 4.57 | w |
| 23.09 | 3.85 | w |
| 23.35 | 3.81 | w |
| 24.12 | 3.69 | m |

Example 4

An 8.0 g portion of the UZM-5 isolated in example 1 was calcined in the following manner. Under a $N_2$ atmosphere, the temperature was ramped to 300°C at 2°C/min, held at 300°C for 2 hr, ramped to 420°C at 2°C/min, held for 2 hr, and ramped to 520°C/min at 2°C/min, and held for 8 hr. An acidic solution was prepared by diluting 40.0 g $HNO_3$ (69%) in 100 g de-ionized water. The solution was heated to 75°C before the calcined UZM-5 was added. The resulting suspension was stirred for 2 hr at 75°C. The product was isolated by filtration, washed with de-ionized water, and dried at 98°C.

Elemental analysis showed the product to have a Si/Al ratio of 89.4, while $N_2$ adsorption measurements indicated a BET surface area of 466 $m^2$/g and a micropore volume of 0.16 cc/g. The x-ray diffraction pattern showed the product to be UZM-5HS, some of the characteristic lines of the pattern are shown in Table 3.

Table 3

| 2-Θ | d(Å) | $I/I_0$ % |
|---|---|---|
| 6.40 | 13.80 | m |
| 7.98 | 11.07 | vs |
| 10.36 | 8.53 | vs |
| 12.12 | 7.30 | vs |
| 14.62 | 6.05 | w |
| 16.00 | 5.53 | s |
| 17.48 | 5.07 | w |
| 19.36 | 4.58 | m |
| 20.74 | 4.28 | w |
| 22.28 | 3.99 | m |
| 22.96 | 3.87 | m |
| 23.27 | 3.82 | m |
| 24.04 | 3.70 | s |

Example 5

A 5 g portion of UZM-5 from example 1 was calcined in the following manner. Under an $N_2$ atmosphere, the temperature was ramped to 550°C at 3°C/min and held there for 6 hr. A solution was prepared by diluting 40 g $HNO_3$ (69%) in 110 g de-ionized water. The solution was heated to 75°C before the calcined UZM-5 was added. The slurry was stirred at 75°C for 4 hr. The product was isolated by filtration, washed with de-ionized water, and dried at 98°C.

Elemental analyses showed the product to have a Si/Al ratio of 60.6, while $N_2$ adsorption measurements indicated a BET surface area of 491 $m^2$/g and a micropore volume of 0.18 cc/g. The x-ray diffraction pattern showed the product to be UZM-5HS, some of the characteristic lines of which are shown in Table 4.

Table 4

| 2-Θ | d(Å) | $I/I_0$ % |
|---|---|---|
| 6.32 | 13.97 | m |
| 7.94 | 11.13 | vs |
| 10.30 | 8.58 | s |
| 12.06 | 7.33 | s - vs |
| 14.65 | 6.04 | w |
| 15.88 | 5.58 | s |
| 17.44 | 5.08 | m |

(continued)

| 2-Θ | d(Å) | $I/I_0$ % |
|---|---|---|
| 19.30 | 4.60 | m |
| 20.76 | 4.28 | w |
| 21.66 | 4.10 | w |
| 22.76 | 3.90 | m |
| 23.14 | 3.84 | w |
| 24.02 | 3.70 | s |

Example 6

A 6 g portion of the UZM-5 from example 1 was calcined by the procedure given in example 3. A solution was prepared by diluting 40g $HNO_3$ (69%) in 60 g de-ionized water. The solution was heated to 75˚C before the addition of the calcined UZM-5. The resulting slurry was stirred for 6 hr at 75˚C. The products were isolated by filtration, washed with de-ionized water, and dried at 98˚C.

Elemental analyses showed the product to have a Si/Al ratio of 117, while $N_2$ adsorption measurements indicated a BET surface area of 489$m^2$/g and a micropore volume of 0.17 cc/g. The x-ray diffraction pattern showed the product to be UZM-5HS with some of the characteristic lines shown in Table 5.

Table 5

| 2-Θ | d(Å) | $I/I_0$ % |
|---|---|---|
| 6.34 | 13.94 | m |
| 7.98 | 11.07 | vs |
| 10.36 | 8.53 | vs |
| 12.12 | 7.30 | s |
| 14.64 | 6.05 | w |
| 15.92 | 5.56 | m-s |
| 17.39 | 5.10 | w |
| 19.28 | 4.60 | m |
| 20.71 | 4.29 | w |
| 22.30 | 3.98 | m |
| 22.88 | 3.88 | m |
| 23.20 | 3.83 | m |
| 24.04 | 3.70 | s |

Example 7

A 10 g portion of a UZM-5 sample (Si/Al = 7.7) was calcined by ramping at 3˚C/min to 560˚C under an $N_2$ atmosphere and held there for 1 hr before changing the atmosphere to air, where the calcination was continued for another 6 hr. A solution was prepared by diluting 60 g $HNO_3$ (69%) in 40 g de-ionized water. The solution was heated to 85˚C before adding the calcined UZM-5. The slurry was stirred for 4 hr at 85˚C. The product was isolated by filtration, washed with de-ionized water, and dried at 95˚C for 1 hr. The dried cake was isolated and treated with a NaCl solution (10 g in 150 ml de-ionized water) at 75˚C for 1 hr. The solid was isolated by filtration, washed with hot de-ionized water, and dried at 95˚C.

Elemental analyses showed the Si/Al ratio to be 317, while $N_2$ adsorption measurements gave a BET surface area of 469 $m^2$/g and a micropore volume of 0.16cc/g. The x-ray diffraction pattern showed the product to be UZM-5HS, some of the characteristic lines are shown in Table 6.

Table 6

| 2-Θ | d(Å) | $I/I_0$ % |
|---|---|---|
| 6.24 | 14.15 | m |
| 7.80 | 11.32 | s |
| 10.22 | 8.65 | vs |
| 11.92 | 7.42 | s |
| 14.49 | 6.11 | w |
| 15.78 | 5.61 | vs |
| 17.41 | 5.09 | w |
| 19.14 | 4.63 | w |
| 22.60 | 3.93 | w-m |
| 22.99 | 3.87 | w |
| 23.90 | 3.72 | m |

Example 8

Aluminum tri-sec-butoxide (95+%), 987.54g was dissolved in 14058g TEAOH (35%) with vigorous stirring. This was followed by the addition of 4954 g colloidal silica (Ludox™ AS-40, 40% $SiO_2$) and further vigorous stirring for a half hour. The reaction mixture was placed in a 22-liter flask equipped with a mechanical stirrer and condensers. The mixture was aged at 95˚C for 16 hr. The reaction mixture contained 4.72% Si after the aging process.

An 1100 g portion of this aluminosilicate reaction mixture was treated with a solution consisting of 6.78 g NaCl and 12.72 g TMACl (97%) dissolved in 150 g de-ionized water with vigorous stirring. After homogenizing for an hour, the resulting mixture was placed in a 2 liter Parr autoclave and digested at 150˚C for 72 hr at autogenous pressures. The product was isolated by filtration and washed with de-ionized water. Characterization by x-ray powder diffraction and elemental analysis showed this material to be UZM-5 of Si/Al ratio 5.88.

A 12 g portion of this UZM-5 was calcined according to the profile given in example 6. A solution was prepared by diluting 60 g $HNO_3$ (69%) in 40 g deionized water. The solution was heated to 85˚C before adding the calcined UZM-5. The slurry was stirred for 4 hr at 85˚C. The product was isolated by filtration, washed with de-ionized water, and dried at 95˚C for 1 hr. The dried cake was isolated and treated with a NaCl solution (10 g NaCl in 150 ml de-ionized water) at 75˚C for 1 hr. The solid was isolated by filtration, washed with hot de-ionized water, and dried at 95˚C.

Elemental analyses showed the Si/Al ratio to be 1479 and Na/Al = 5.48, while $N_2$ adsorption measurements gave a BET surface area of 512 $m^2/g$ and a micropore volume of 0.18 cc/g. The x-ray diffraction pattern showed the product to be UZM-5HS, some of the characteristic lines are shown in Table 7.

Table 7

| 2-Θ | d(Å) | $I/I_0$ % |
|---|---|---|
| 6.38 | 13.83 | w |
| 7.95 | 11.12 | vs |
| 10.32 | 8.56 | s |
| 12.10 | 7.31 | vs |
| 14.59 | 6.07 | w |
| 15.96 | 5.55 | m |
| 16.68 | 5.31 | w |
| 17.43 | 5.08 | w |
| 19.12 | 4.64 | w-m |
| 20.75 | 4.28 | w |

(continued)

| 2-Θ | d(Å) | $I/I_0$ % |
|---|---|---|
| 22.33 | 3.98 | w |
| 23.24 | 3.82 | m |
| 24.05 | 3.70 | m |

Example 9

A sample of UZM-5 (Si/Al = 6.03) was pretreated at 300˚C for 10 hr under an $N_2$ atmosphere. The UZM-5 sample (3.6 g) was suspended in 30 g deionized water containing 3 g $NH_4NO_3$ and 9.59 g $HNO_3$ (69%), giving a 15/1 ratio of $HNO_3$/Al. The slurry was heated overnight at 80˚C with stirring in an oil bath. The solids were isolated by filtration, washed with de-ionized water, and dried at room temperature. The sample was then calcined at 500˚C in air.

Elemental analyses showed the Si/Al ratio to be 65.4, while $N_2$ adsorption measurements gave a BET surface area of 498 $m^2$/g and a micropore volume of 0.19 cc/g. The x-ray diffraction pattern showed the product to be UZM-5HS, some characteristic lines given in Table 8.

Table 8

| 2-Θ | d(Å) | $I/I_0$ % |
|---|---|---|
| 6.43 | 13.74 | w |
| 8.02 | 11.01 | m |
| 10.42 | 8.48 | m |
| 12.16 | 7.27 | vs |
| 16.00 | 5.53 | s |
| 17.31 | 5.12 | w |
| 19.28 | 4.60 | w |
| 22.30 | 3.98 | w |
| 23.23 | 3.83 | w |
| 24.12 | 3.69 | m-s |

Example 10

A sample of the UZM-5 prepared in example 9 (6.37g, Si/Al = 6.03, 21.3% volatiles) was suspended in a $HNO_3$ solution containing 32.4 g $HNO_3$ (69%) diluted with 50 g de-ionized water, giving a 30/1 ratio of $HNO_3$/Al. The slurry was heated 18 hr at 80˚C with stirring in an oil bath. The product was isolated by filtration, washed with de-ionized water, and dried at room temperature. The sample was then calcined for 4 hr at 500˚C in air.

Elemental analyses showed the Si/Al ratio to be 23.2, while $N_2$ adsorption measurements gave a BET surface area of 510 $m^2$/g and a micropore volume of 0.20 cc/g. The x-ray diffraction pattern showed the product to be UZM-5HS, some characteristic lines are given in Table 9.

Table 9

| 2-Θ | d(Å) | $I/I_0$ % |
|---|---|---|
| 6.43 | 13.74 | w |
| 7.92 | 11.15 | m |
| 10.30 | 8.58 | m |
| 12.08 | 7.32 | vs |
| 15.98 | 5.54 | vs |
| 19.19 | 4.62 | w |

(continued)

| 2-Θ | d(Å) | $I/I_0$ % |
|---|---|---|
| 20.76 | 4.28 | w |
| 22.52 | 3.94 | w |
| 23.21 | 3.83 | w |
| 24.02 | 3.70 | m |

Example 11

An ammonium hexafluorosilicate solution was prepared by dissolving 8.37 g $(NH_4)_2SiF_6$ in 159 g de-ionized water. Separately, an ammonium exchanged UZM-5 sample (150.55 g, Si/Al = 5.45) was suspended in 395 g deionized water and heated to 80˚C. The ammonium hexafluorosilicate solution was then added to the zeolite slurry at a rate of 1.34 cc/min using a pump over a period of 120 minutes. Once the addition was completed, the resulting reaction mixture was held at 80˚C for an hour. The product was isolated by filtration, washed with de-ionized water, and dried at room temperature.

Elemental analyses showed the Si/Al ratio to be 8.25, while $N_2$ adsorption showed the BET surface area to be 561 $m^2/g$ and the micropore volume to be 0.17 cc/g. The x-ray diffraction pattern showed the product to be UZM-5HS, some characteristic lines are given in Table 10.

Table 10

| 2-Θ | d(Å) | $I/I_0$ % |
|---|---|---|
| 6.02 | 14.68 | w |
| 7.69 | 11.49 | s |
| 10.28 | 8.60 | s |
| 11.94 | 7.41 | vs |
| 15.84 | 5.59 | m-s |
| 16.48 | 5.37 | w |
| 19.07 | 4.65 | w |
| 20.63 | 4.30 | w |
| 22.12 | 4.02 | m |
| 22.40 | 3.97 | m |
| 23.07 | 3.85 | w |
| 23.86 | 3.73 | vs |

## Claims

**1.** A microporous crystalline zeolite having a three-dimensional framework of at least $AlO_2$ and $SiO_2$ tetrahedral oxide units and an empirical composition on an anhydrous basis in terms of mole ratios of the elements of:

$$M1_a^{n+} Al_{(1-x)}E_xSi_{y'}O_{z''}$$

where M1 is at least one exchangeable cation selected from the group consisting of alkali metals, alkaline earth metals, rare earth metals, ammonium ion, hydrogen ion and mixtures thereof, a is the mole ratio of M1 to (A1 + E) and varies from 0.15 to 5.0, E is an element selected from the group consisting of gallium, iron, boron, chromium, indium and mixtures thereof, x is the mole fraction of E and varies from 0 to 0.5, n is the weighted average valence of M1 and has a value of +1 to +3, y' is the mole ratio of Si to (A1 + E) and is greater than 5 and z" is the mole ratio of O to (A1 + E) and has a value determined by the equation:

$$Z'' = (a \bullet n + 3 + 4 \bullet y')/2$$

the zeolite **characterised in that** it has at least two x-ray diffraction peaks, one at a *d*-spacing of 3.84±0.07Å and one at a *d*-spacing of 8.55±0.25Å, and further **characterized in that** it has an x-ray powder diffraction pattern which contains at least the d-spacing and relative intensities given in the Table below:

**Table A**

| UZM-5HS | | |
|---|---|---|
| 2-Θ | d(Å) | $I/I_0$% |
| <6.79 | >13.0 | w-m |
| 8.26 - 7.52 | 10.70 - 11.75 | m-vs |
| 10.65 - 10.04 | 8.30 - 8.80 | m-vs |
| 12.32 - 11.79 | 7.18 - 7.50 | s-vs |
| 16.56 - 15.53 | 5.35 - 5.70 | m-vs |
| 19.71 - 18.78 | 4.50 - 4.72 | w-m |
| 23.58 - 22.72 | 3.77 - 3.91 | w-m |
| 24.37 - 23.64 | 3.65 - 3.76 | m-vs |

**2.** The zeolite of claim 1 where y' has a value from 5 to 20,000

**3.** A hydrocarbon conversion process comprising contacting a hydrocarbon stream with a catalytic composite at hydrocarbon conversion conditions to give a converted product, the catalytic composite comprising the zeolite of claims 1 or 2.

**4.** The process of claim 3 where the hydrocarbon conversion process is selected from the group consisting of alkylation of aromatics, isomerization of xylenes, naphtha cracking, ring opening, transalkylation, alkylation of isoparaffins, isomerization of ethyl benzene and conversion of oxygenates to olefins.

## Patentansprüche

**1.** Mikroporöser kristalliner Zeolith mit einem dreidimensionalen Gerüst von mindestens $AlO_2$- und $SiO_2$-Oxidtetraedereinheiten und der folgenden empirischen Zusammensetzung auf wasserfreier Basis in Form der Molverhältnisse der Elemente:

$$M1_a^{n+}Al_{(1-x)}E_xSi_{y'}O_{z''}$$

worin M1 für mindestens ein austauschbares Kation aus der Gruppe bestehend aus Alkalimetallen, Erdalkalimetallen, Seltenerdmetallen, Ammoniumion, Wasserstoffion und Mischungen davon steht, a für das Molverhältnis von M1 zu (A1 + E) steht und von 0,15 bis 5,0 variiert, E für ein Element aus der Gruppe bestehend aus Gallium, Eisen, Bor, Chrom, Indium und Mischungen davon steht, x für den Molenbruch von E steht und einen Wert von 0 bis 0,5 aufweist, n für die gewichtete durchschnittliche Wertigkeit von M1 steht und einen Wert von +1 bis +3 aufweist, y' für das Molverhältnis von Si zu (A1 + E) steht und größer als 5 ist und z" für das Molverhältnis von O zu (A1 + E) steht und einen durch die folgende Formel bestimmten Wert aufweist:

$$Z'' = (a \cdot n + 3 + (4 \cdot y')/2$$

**dadurch gekennzeichnet, daß** er mindestens zwei Röntgenbeugungspeaks aufweist, einen bei einem d-Abstand

von 3,84 ± 0,07 Å und einen bei einem d-Abstand von 8,55 ± 0,25 Å, und ferner **dadurch gekennzeichnet, daß** er ein Röntgenpulverbeugungsmuster aufweist, das mindestens die in der nachstehenden Tabelle angegebenen d-Abstände und relativen Intensitäten enthält:
Tabelle A

| UZM-5HS | | |
|---|---|---|
| 2-θ | d (Å) | $I/I_0$ % |
| <6,79 | >13,0 | w-m |
| 8,26 - 7,52 | 10,70 - 11,75 | m-vs |
| 10,65 - 10,04 | 8,30 - 8,80 | m-vs |
| 12,32 - 11,79 | 7,18 - 7,50 | s-vs |
| 16,56 - 15,53 | 5,35 - 5,70 | m-vs |
| 19,71 - 18,78 | 4,50 - 4,72 | w-m |
| 23,58 - 22,72 | 3,77 - 3,91 | w-m |
| 24,37 - 23,64 | 3,65 - 3,76 | m-vs |

**2.** Zeolith nach Anspruch 1, wobei y' einen Wert von 5 bis 20.000 aufweist.

**3.** Kohlenwasserstoffumwandlungsverfahren, bei dem man einen Kohlenwasserstoffstrom unter Kohlenwasserstoffumwandlungsbedigungen mit einem katalytisch wirksamen Verbund in Berührung bringt, wobei man ein umgewandeltes Produkt erhält, wobei der katalytisch wirksame Verbund den Zeolith nach Anspruch 1 oder 2 umfaßt.

**4.** Verfahren nach Anspruch 3, bei dem das Kohlenwasserstoffumwandlungsverfahren aus der Gruppe bestehend aus Alkylierung von Aromaten, Isomerisierung von Xylolen, Naphtha-Cracking, Ringöffnung, Umalkylierung, Alkylierung von Isoparaffinen, Isomerisierung von Ethylbenzol und Umwandlung von Oxygenaten in Olefine ausgewählt wird.

## Revendications

**1.** Zéolite cristalline microporeuse possédant une structure tridimensionnelle d'au moins des unités d'oxydes tétraédriques $AlO_2$ et $SiO_2$ et une composition empirique sur une base anhydre exprimée en rapports molaires des éléments de :

$$Ml_a^{n+}Al_{(1-x)}E_xSi_{y'}O_{z''}$$

dans laquelle M1 représente au moins un cation échangeable choisi parmi le groupe constitué de métaux alcalins, de métaux alcalino-terreux, de métaux de terres rares, de l'ion ammonium, de l'ion hydrogène et de mélanges de ceux-ci, a représente le rapport molaire de M1 à (A1 + E) et varie de 0,15 à 5,0, E représente un élément choisi parmi le groupe constitué du gallium, du fer, du bore, du chrome, de l'indium et de mélanges de ceux-ci, x représente la fraction molaire de E et varie de 0 à 0,5, n représente la valence moyenne pondérée de M1 dont la valeur varie de +1 à +3, y' représente le rapport molaire de Si à (A1 + E) et est supérieur à 5 et z" représente le rapport molaire de O à (A1 + E) dont la valeur est déterminée par l'équation :

$$z'' = (a \bullet n + 3 + 4 \bullet y')/2$$

la zéolite est **caractérisée en ce qu'**elle possède au moins deux pics de diffraction aux rayons X, un à un espace d de 3,84 ± 0,07 Å et un à un espace d de 8,55 ± 0,25 Å, et **caractérisée en outre en ce qu'**elle possède un spectre de diffraction aux rayons X sur poudre qui contient au moins l'espace d et les intensités relatives donnés

dans le tableau ci-dessous :

Tableau A

| UZM-5HS | | |
|---|---|---|
| 2-Θ | d(Å) | $I/I_0$% |
| <6,79 | >13,0 | w-m |
| 8,26 - 7,52 | 10,70 - 11,75 | m-vs |
| 10,65 - 10,04 | 8,30 - 8,80 | m-vs |
| 12,32 - 11,79 | 7,18 - 7,50 | s-vs |
| 16,56 - 15,53 | 5,35 - 5,70 | m-vs |
| 19,71 - 18,78 | 4,50 - 4,72 | w-m |
| 23,58 - 22,72 | 3,77 - 3,91 | w-m |
| 24,37 - 23,64 | 3,65 - 3,76 | m-vs |

**2.** Zéolite selon la revendication 1, dans laquelle y' a une valeur de 5 à 20 000.

**3.** Procédé de conversion d'hydrocarbures comprenant la mise en contact d'un flux d'hydrocarbures avec un composite catalytique dans des conditions de conversion d'hydrocarbures pour obtenir un produit transformé, le composite catalytique comprenant la zéolite des revendications 1 ou 2.

**4.** Procédé selon la revendication 3, dans lequel le procédé de conversion d'hydrocarbures est choisi parmi le groupe constitué de l'alkylation d'hydrocarbures aromatiques, de l'isomérisation de xylènes, du craquage de naphta, de l'ouverture de cycles, de la transalkylation, de l'alkylation d'isoparaffines, de l'isomérisation de l'éthylbenzène et de la conversion de composés oxygénés en oléfines.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4996034 A
- US 4503023 A
- US 20030211034 A
- US 6613302 B1
- US 6388159 B1
- US 4310440 A
- US 4440871 A
- US 5015796 A


### Non-patent literature cited in the description


- **D.W. Breck.** Zeolite Molecular Sieves. John Wiley and Sons, 1974, 636
- **H. Pines.** THE CHEMISTRY OF CATALYTIC HYDROCARBON CONVERSIONS. Academic Press, 1981, 123-154